# EUROPÄISCHE PATENTSCHRIFT

(11) **EP 2 138 140 B1**
(45) Veröffentlichungstag und Bekanntmachung des Hinweises auf die Patenterteilung: **19.03.2014**
(21) Anmeldenummer: 09008203.3
(22) Anmeldetag: 23.06.2009
(51) Int. Cl.: A61F 13/08

(54) **Segmental zirkulär adaptive Kompressionsmanschette**
Segmentally circular adaptive compression cuff
Manchette de compression adaptable circulairement et segmentairement

(30) Priorität: 25.06.2008 DE 202008008422 U
(43) Veröffentlichungstag der Anmeldung: 30.12.2009
(73) Patentinhaber: Hechmat, Abolgh, 55122 Mainz (DE)
(72) Erfinder: Hechmat, Abolgh, 55122 Mainz (DE)
(74) Vertreter: Kodron, Felix

(56) Entgegenhaltungen:
- WO-A1-01/72250
- DE-A1- 2 736 381
- DE-U1- 20 014 336
- US-A- 3 856 008
- US-A- 5 520 630

## Beschreibung

Die Erfindung betrifft eine segmental zirkulär adaptive Kompressionsmanschette zur humanmedizinischen Behandlung des Varicosesymptomkomplexes im Bereich der Beine, bestehend aus einem längsgeschlitzten elastischen Kompressionsstrumpf und einer Vielzahl von die Längsschlitzränder lösbar und mit variabler Spannkraft miteinander verbindenden, handbetätigbaren Verschlüssen.

Herkömmliche, rundum geschlossene Kompressionsstrümpfe, werden entweder in Standardgrößen produziert oder einzeln individuell auf die spezielle Beinform des Patienten angepaßt und hergestellt.

Das An- und Ausziehen dieser bekannten herkömmlichen Kompressionsstrümpfe ist oftmals ausgesprochen mühsam und insbesondere von wenig beweglichen Patienten kaum zu bewältigen.

Auch ist der Verschleiß des elastischen Strumpfmaterials durch das kraftaufwendige An- und Ausziehen des Kompressionsstrumpfes hoch, so dass die Lebensdauer eines herkömmlichen Kompressionsstrumpfes beschränkt ist. Der Verschleiß und das Ausleiern des Strumpfgewebes ist insbesondere im Knöchelbereich sehr ungünstig, da es gerade dort keine natürliche Muskelpumpe gibt, die das Blut kopfwärts transportiert.

Auch kann mittels herkömmlicher Kompressionsstrümpfe nur ein gleichmäßiger homogener Druck im gesamten Strumpfbereich erzielt werden, jedoch keine Anpassung des Kompressionsdruckes an unterschiedlichen Beinbereichen eingestellt werden.

Durch das in alle Richtungen elastische Strumpfgewebe kommt es ferner regelmäßig zu unerwünschter Faltenbildungen z.B. im Bereich der Kniekehlen.

Aus der DE 42 30 165, die auf den Anmelder zurückgeht, ist eine längsgeschlitzte Kompressionsmanschette bekannt, die entlang der Längsschlitzränder auf der einen Seite zahlreiche schmale Klettbänder aufweist, die durch jeweils einen an der gegenüberliegenden Seite angeordneten Ring gezogen werden.

Der Längsschlitz ist hierbei auf der Beinrückseite angeordnet. Die Handhabung der auf der Beinrückseite angeordneten Verschlüsse ist jedoch für den Patienten beschwerlich.

Aus der DE 44 19 287, die ebenfalls auf den Anmelder zurückgeht, ist eine Kompressionsmanschette bekannt, die mittels Verschlußringen an einem Rand des Längsschlitzes und Klettbändern an der gegenüberliegenden Seite verschlossen wird, wobei die Verschlußringe im Knöchelbereich des Fußgelenkes auf einer Seite des Fußes und im übrigen Beinbereich auf der Vorderseite des Beines angeordnet sind.

Aus der EP 95118756.6 ist ein längsgeschlitzter Kompressionsstrumpf bekannt, bei welchem der Längsschlitz derart beschaffen ist, dass der Schlitzverlauf im oberen Beinabschnitt entlang der Vorderseite des Beines verläuft, jedoch im Knöchelbereich seitlich bis zu den Fußzehen verläuft. Weiterhin sind die Verschlüsse im Beinoberbereich bis etwa zehn mal breiter bemessen als im Beinunterbereich.

Auch bei dieser erfinderischen Lösung kann die am Bein anliegende strumpfförmige Kompressionsmanschette bei gelösten Verschlüssen vollständig aufgeklappt und um das Bein des Patienten herum gelegt werden.

Anschließend werden die Verschlüsse einzeln mittels Klettbändern und Ringen mit variabler Spannkraft geschlossen.

Nachteilig bei dieser bekannten Kompressionsmanschette ist jedoch, dass die Verschlüsse im oberen Beinbereich breiter bemessen sind als im unteren Beinbereich und dadurch der Kompressionsdruck nur ungenau nach Gefühl eingestellt werden kann. Weiterhin ist es nachteilig, dass die Verschlüsse im Knöchelbereich an einer Seite des Fußes angeordnet sind und dadurch bei Bewegung des Fußes gegeneinander drücken und sich durch die Verschlußringe wechselseitig behindern.

Bei allen vorbenannten Kompressionsmanschetten ist es weiterhin nachteilig, dass die Zugrichtung der Verschlüsse nur in eine Richtung erfolgt, so dass es beim Anlegen der Kompressionsmanschette zum allmählichen verrutschen der Kompressionsmanschette kommt.

Aus der WO 01/72250 A1 ist eine mehrteilige Kompressionsmanschette mit einer separaten, großen Zunge entlang des Schienbeines bekannt, die entlang ihrer Längsschnittränder vorderseitig im oberen Beinabschnitt wenige, jeweils paarweise angeordnete, identische geformte Verschlusslaschen aufweist, die jeweils durch eine gemeinsame metallene Verschlussklammer auf der separaten, auf dem Schienbein aufliegenden Zunge geführt und durch Rückzug zur jeweils eigenen Seite fixiert werden.

Im unteren Beinabschnitt im Bereich des Knöchels und des Fusses sind die Verschlusslaschen zwar noch immer gegenüberliegend angeordnet, jedoch jetzt verschieden geformt, so dass sie ohne eine metallene Verschlusskammer auf der Zunge durch einfaches Durchstecken der einen kleineren Lasche durch eine Durchbrechung in der gegenüberliegenden größeren Lasche und Fixierung beider Laschen durch Zug an beiden Laschen zur jeweils gegenüberliegenden Seite der Kompressionsmanschette mittels Klettverschluss geschlossen werden.

In der US 5,529,630 ist eine Kompressionsmanschette offenbart, die nur entlang des Beines verläuft und nicht den Knöchel- und Fußbereich umfasst und entlang der Wade rückseitig verschlossen wird. Die Kompressionsmanschette wird dabei über einen großflächig entlang der Längsschlitzränder angeordneten Klettverschluss geschlossen. Um die Handhabbarkeit zu verbessern, ist eine Bauform mit vollständig alternierend angeordneten Klettverschlusslaschen entlang der Wade offenbar mit einer Anordnung der Verschlüsse entlang der Beinrückseite. Diese Kompressionsmanschette ist vor allem für Schwangere oder solche Patienten gedacht, bei denen durch die Kompressionsmanschette lediglich ein genereller Druck auf das Bein ausgeübt werden soll.

Aus der US-Schrift 3,856,008 ist schließlich ein geschlossener Kompressionsstrumpf für lange Tierbeine bekannt, der einen besonders hohen Druck über die gesamte Beinlänge ausüben soll. An seiner Vorseite weist er zwei große Öffnungen auf, deren Ränder mittels zahlreicher, alternierend angeordneter Klettverschlusslaschen miteinander verbunden werden können. Über dem Knie ist eine geschlossene Materialbrücke angeordnet. Dieser offenbarte spezielle Hochdruck-Kompressionsstrumpf für lange Tierbeine ist für eine Anwendung am menschlichen Bein nicht vorgesehen.

Es ist die Aufgabe der vorliegenden Erfindung, eine segmental zirkulär adaptive Kompressionsmanschette zu schaffen, bei welcher die Handhabung für den Patienten einfach gehalten wird, der Kompressionsdruck in den einzelnen Beinbereichen bei geringem Kraftaufwand variabel einstellbar ist, die außerdem am Bein des Patienten ohne zu Verrutschen gut sitzt und auch im lockeren Schuh getragen werden kann.

Gelöst wird diese Aufgabe nach der Erfindung durch eine segmental zirkulär adaptive Kompressionsmanschette mit den Merkmalen des Anspruchs 1.

Dabei sind die Verschlüsse entlang der Längsschlitzränder im oberen Bereich der Kompressionsmanschette oberhalb des Knöchels nur einseitig angeordnet und im unteren Bereich der Kompressionsmanschette alternierend zueinander angeordnet, und die Zugrichtung der Verschlüsse ist im oberen Bereich der Kompressionsmanschette jeweils nur zu einer Seite hin ausgerichtet, während die Zugrichtung der Verschlüsse im unteren Bereich der Kompressionsmanschette alternierend zueinander ausgerichtet ist, wobei die Kompressionsmanschette in ihrer Längsausrichtung nicht-elastisch und nur in ihrer Querausrichtung elastisch ausgebildet ist.

Dadurch kann im oberen Beinbereich ohne große Kraftanstrengung ein relativ großer zirkulärer Kompressionsdruck an jedes Segment angelegt werden, im unteren Bereich, insbesondere entlang des Fusses, wird jedoch durch die alternierende Anordnung der Verschlüsse zueinander ein einseitiger Zug vermieden. Dadurch wird verhindert, dass die angelegte Kompressionsmanschette in ihrer Längsrichtung verrutscht und auch gewährleistet, dass der Abstand der Verschlußssegmente zueinander gleich bleibt.

Der segmentale zirkuläre Kompressionsdruck wird bei nur querelastischen Fasern besonders wirksam, da ein geschlossenes Druckfeld am Bein entsteht ohne Drucklücken zwischen den einzelnen Segmenten.

Bei einer besonders vorteilhaften Ausführung der Erfindung wird der Kompressionsdruck an mindestens einem Segment der Kompressionsmanschette mittels eines Zugkraftmessgerätes und/oder Drucksensors gemessen, so dass in mindestens einem von der Kompressionsmanschette umschlossenen Beinbereich ein genau definierter Kompressionsdruck auf den Verschluss gelegt werden kann, um eine optimale Anpassung der Kompressionsmanschette an mindestens einem Segment an das Bein zu erreichen und den Wirkungsgrad zu optimieren.

Die erfindungsgemäße segmental zirkulär adaptive Kompressionsmanschette wird zunächst um das Bein des Patienten herumgelegt und der oberste Verschluss um das Bein herum locker geschlossen. Die Kompressionsmanschette soll den ganzen Fuß umschließen können.

Nun wird das unterste Klettband oberhalb des Sprunggelenks fixiert. Dann beginnt man, die vordersten, im Fußbereich liegenden Verschlüsse zu schließen und zu adaptieren. Die Verschlüsse liegen im Fußbereich jeweils abwechselnd mal auf dem einen Längsschlitzrand der Kompressionsmanschette, mal auf der anderen Seite, so dass die Verschlüsse mal von links nach rechts, dann wieder von rechts nach links über die Fußoberseite verschlossen werden (Gegenzug).

Zum verbesserten Adaptieren ist am jeweiligen Verschlussende ein z.B. digitales Zugmessgerät befestigt und/oder wird ein Drucksensor unter den Verschluss gelegt, so dass der Verschluss derart fest um das Bein des Patienten gelegt werden kann, bis ein vom Arzt gewünschter Kompressionsdruck erreicht ist. So wird segmental von Verschluss zu Verschluss von unten nach oben zirkulär der Kompressionsdruck mit abnehmendem Druck zur Körpermitte hin adaptiert. Jedes Segment der Kompressionsmanschette ist funktionsmäßig unabhängig vom benachbarten Segment, insgesamt jedoch bilden alle Segmente eine funktionale Einheit.

Liegen im Beinbereich Zonen vor, die nicht oder nur gering unter Kompressionsdruck gestellt werden dürfen, z.B. bei Hautverletzungen durch Beinödeme, ist es möglich, in diesem Abschnitt nur einen geringen Kompressionsdruck am entsprechenden Verschluss der Kompressionsmanschette einzustellen, um eine bessere und schnellere Wundheilung zu erreichen.

Vorteilhafterweise ist jeder Verschluss mit einer Skala versehen, die es dem Patienten möglich macht, die vom Arzt vorgenommene erste Einstellung selbstständig auch ohne Zugmessgerät und/oder Drucksensor zu reproduzieren.

Bei einer anderen vorteilhaften Ausführungsform der Erfindung sind an der Beinrückseite der Kompressionsmanschette außenseitig weitere Befestigungsmittel für die Verschlüsse angeordnet, beispielsweise ein in Längsrichtung verlaufender Klettstreifen, so dass überstehende Verschlüsse bei besonders eng um das Bein gelegter Kompressionsmanschette auf der Beinrückseite nochmals fixiert werden.

Bei einer anderen Ausführungsform der Erfindung besteht die Kompressionsmanschette aus einem lösbar miteinander verbundenen Beinteil und einem Fußteil, also aus zwei separaten, miteinander lösbar verbundenen Teilen, um einen besseren Tragekomfort in Schuhen zu erreichen oder um Wundbereiche im Knöchelbereich auszusparen.

Das Fußteil kann hierbei als offene Manschette oder auch als geschlossener Strumpf vorliegen.

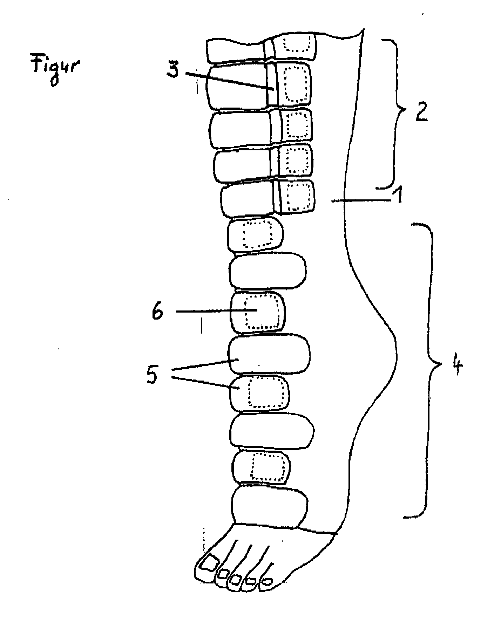

Die erfindungsgemäße Kompressionsmanschette ist in Figur 1 näher erläutert und beschrieben.

Figur 1 zeigt die Kompressionsmanschette 1 am Bein eines Patienten in geschlossenem Zustand, wobei die Verschlüsse aus Klettbändern 5 bestehen, die entweder durch einen Verschlussring 3 auf der gegenüberliegenden Seite gezogen oder auf einem gegenüberliegenden Klettfeld 6 befestigt werden.

Im Beinbereich 2 oberhalb des Knöchels sind die Klettbänder 5 der Verschlüsse alle auf einer Seite des Längsschlitzrandes angeordnet, und die zugehörigen Verschlussringe 3 entlang des gegenüberliegenden Längsschlitzrandes.

Im Knöchel- und Fußbereich 4 der Kompressionsmanschette 1 sind die Klettbänder 5 wechselseitig angebracht und werden auf einem jeweils gegenüberliegenden Klettfeld 6 fixiert, so dass im Knöchel- und Fußbereich 4 die Kompressionsmanschette 1 durch den Verzicht auf die Verschlussringe 3 wesentlich dünner ausfällt als im oberen Beinbereich 2 und damit noch ein lockerer Schuh angezogen werden kann. Durch den Gegenzug im Fußbereich zahlreicher schmaler Verschlüsse wird weiterhin ein besonders guter segmental zirkulärer Druck in diesem Bereich einstellbar, was besonders wichtig ist, da eine natürliche Muskelpumpe im Knöchel- und Fußbereich fehlt, und es leicht zu Stauungen kommt.

## Patentansprüche

1. Segmental zirkulär adaptive Kompressionsmanschette (1) bestehend aus einem längsgeschlitzten elastischen Strumpf und einer Vielzahl von die Längsschlitzränder lösbar und mit variabler Spannkraft verbindenden, handbetätigbaren Verschlüssen, wobei Verschlüsse entlang der Längsschlitzränder der Kompressionsmanschette (1) alternierend zueinander angeordnet sind,
**dadurch gekennzeichnet, dass**
- die Verschlüsse als Klettbänder (5) im Beinbereich (2) oberhalb des Knöchels nur auf einer Seite des Längsschlitzrandes und zugehörige Verschlussringe (3) entlang des gegenüberliegenden Längsschlitzrandes angeordnet sind
- und im Fussbereich (4) der Kompressionsmanschette(1) die Verschlüsse als Klettbänder (5) wechselseitig angebracht und auf einem jeweils gegenüberliegenden Klettfeld (6) fixierbar sind,
- wobei die Zugrichtung der Verschlüsse im oberen Bereich (2) der Kompressionsmanschette (1) jeweils nur zu einer Seite hin ausgerichtet ist
- und die Zugrichtung der Verschlüsse im unteren Bereich (4) der Kompressionsmanschette (1) alternierend zueinander ausgerichtet ist und
- die Kompressionsmanschette (1) in ihrer Längsrichtung nicht-elastisch und nur in Querrichtung elastisch ausgebildet ist.

2. Segmental zirkulär adaptive Kompressionsmanschette (1) nach Patentanspruch 1,
**dadurch gekennzeichnet, dass**
der Kompressionsdruck an mindestens einem Segment der Kompressionsmanschette (1) mittels eines Zugkraftmessgerätes und/oder eines Drucksensors messbar ist.

3. Segmental zirkulär adaptive Kompressionsmanschette (1) nach Patentanspruch 1,
**dadurch gekennzeichnet, dass**
an den Verschlüssen eine Skala angebracht ist zur wiederholbaren Einstellung eines definierten Kompressionsdrucks.

4. Segmental zirkulär adaptive Kompressionsmanschette (1) nach einem der vorangegangenen Patentansprüche,
**dadurch gekennzeichnet, dass**
auf der Kompressionsmanschette (1) im Bereich der Beinrückseite außenseitig weitere Befestigungsmittel für die Verschlüsse angeordnet sind.

5. Segmental zirkulär adaptive Kompressionsmanschette (1) nach einem der vorangegangenen Patentansprüche,
**dadurch gekennzeichnet, dass**
die Kompressionsmanschette (1) aus einem lösbar miteinander verbundenen Beinteil und einem Fußteil besteht.

## Claims

1. Segmental circular adaptive compression sleeve (1) consisting of an elastic stocking with longitudinal slit and a number of connecting, manually operated closures of varying tension force, which can be released from the edges of the longitudinal slits whereby the closures along the edges of the longitudinal slits of the compression sleeve (1) are positioned alternatively **characterized in that**
- the closures in the form of hook-and-loop fastenerstripes (5) in the leg area (2) above the ankle are only on one side of the edge of the longitudinal slit and the corresponding closure rings(3) are positioned on the opposite side of the longitudinal slit
- and in the foot area(4) of the compression sleeve (1) the hook-and-loop fastenerstripes closures (5) are positioned on alternating sides and affixed to the field of fastener loops on the opposite side
- whereby the tension direction of the closure in the upper area (2) of the compression sleeve (1) is only set in one direction
- and the direction of tension of the closures in the lower area (4) of the compression sleeve (1) is set in alternating directions to one another and
- the compression sleeve is not elastic in the longitudinal direction and is only elastic in the cross direction

2. Segmental circular adaptive compression sleeve (1) in accordance with patent claim 1
**characterized in that**
the compression pressure in at least one segment of the compression sleeve (1) can be measured by means of a tensile force measuring device /and/or pressure sensor

3. Segmental circular adaptive compression sleeve (1) in accordance with patent claim 1
**characterized in that**
a scale is incorporated in the closures for repeat setting of a specified compression pressure.

4. Segmental circular adaptive compression sleeve (1) in accordance with one of the foregoing patent claims
**characterized in that**
on the compression sleeve in the leg area on the outside, further fastening features are included for the closures.

5. Segmental circular adaptive compression sleeve (1) in accordance with one of the foregoing patent claims
**characterized in that**
the compression sleeve (1) consists of a leg section connected to a foot section which are separable

## Revendications

1. Manchette de compression adaptative circulaire segmentale (1) se composant d'une chaussette élastique à fente longitudinale et d'une multitude de fermetures actionnables manuellement, détachables des bordures de fente longitudinale et reliant avec une force de tension variable, les fermetures étant disposées en alternance l'une par rapport à l'autre le long des bordures de fente longitudinale de la manchette de compression (1),
**caractérisée en ce que**
- les fermetures sont disposées seulement sur un côté de la bordure de fente longitudinale comme bandes autoagrippantes (5) dans la zone de la jambe (2) au-dessus de la cheville et des boucles de fermetures associées (3) le long de la bordure de fente longitudinale opposée
- et dans la zone du pied (4) de la manchette de compression (1) les fermetures sont disposées en alternance comme bandes autoagrippantes (5) et fixables sur un champ de bardane se trouvant respectivement en face (6),
- le sens de traction des fermetures étant orienté dans la zone supérieure (2) de la manchette de compression (1) respectivement seulement vers un côté,
- le sens de traction des fermetures étant orienté dans la zone inférieure (4) de la manchette de compression (1) en alternance l'une par rapport à l'autre, et
- la manchette de compression (1) étant constituée de manière non-élastique dans sa direction longitudinale et seulement de manière élastique dans sa direction transversale.

2. Manchette de compression adaptative circulaire segmentale (1) selon la revendication 1,
**caractérisée en ce que**
la pression de compression est mesurable sur au moins un segment de la manchette de compression (1) au moyen d'un dynamomètre et/ou d'un capteur de pression.

3. Manchette de compression adaptative circulaire segmentale (1) selon la revendication 1,
**caractérisée en ce que**
sur les fermetures, une graduation est apposée en vue du réglage répétable d'une pression de compression définie.

4. Manchette de compression adaptative circulaire segmentale (1) selon une quelconque des revendications précédentes,
**caractérisée en ce que**
sur la manchette de compression (1) dans la zone du dos de la jambe, d'autres moyens de fixation sont apposés à l'extérieur pour les fermetures.

5. Manchette de compression adaptative circulaire segmentale (1) selon une quelconque des revendications précédentes,
**caractérisée en ce que**
la manchette de compression (1) se compose d'une partie de jambe et d'une partie de pied reliées entre elles de manière amovible.
